# EUROPEAN PATENT APPLICATION

(11) **EP 2 380 632 A1**
(43) Date of publication of application: **26.10.2011**
(21) Application number: 11174829.9
(22) Date of filing: 16.01.2007
(51) Int. Cl.: A61P 27/02, A61K 31/13, A61K 31/4168, A61K 31/498, A61K 31/195, A61K 31/485

(54) **The use of NMDA antagonists to attenuate vitreoretinal vascular endothelial growth factor (VEGF) protein levels in animals**

(30) Priority: 17.01.2006 US 759905 P
(62) Divisional of application: 07716650.2
(71) Applicant: Allergan, Inc., Irvine, CA 92612 (US)
(72) Inventor: Kusari, Jyotimoy X., Irvine, CA California 92603 (US); Gil, Daniel W., Corona Del Mar, CA California 92625 (US)
(74) Representative: HOFFMANN EITLE

(57) **Abstract**

Reducing the VEGF level in a patient suffering from an ocular disease or condition which is characterized by elevated VEGF and/or blood retina barrier (BRB) breakdown which comprises treating said patient with an effective amount of a compound selected from the group consisting of memantine, brimonidine and mixtures thereof.

## Description

### CROSS REFERENCE TO RELATED APPLICATIONS

This application is based on, and claims the benefit of, U.S. Provisional Application No. 60/759,905, filed January 17, 2006, and which is incorporated herein by reference.

### BACKGROUND OF THE INVENTION

### I. Field of the Invention

This invention relates to the use of memantine and/or brimonidine or other Alpha 2 adrenergic agonists to control VEGF levels in an animal.

### 2. Description of the Art

Vascular endothelial growth-factor (VEGF) is one of the most potent inducers of vascular permeability and is a powerful mitogen for endothelial cells. Recent evidence has suggested that VEGF may play a role in the pathogenesis of neovascularization including proliferative diabetic retinopathy (PDR) and age-related Macular degeneration (AMD), and in the increase of vascular permeability that characterizes early stages of diabetic retinopathy, tumors, wound healing and inflammatory conditions.

Various stimuli relevant to diabetic retinopathy have been reported to increase the vascular expression of VEGF, including hypoxia, an elevated glucose concentration, activation of protein kinase C (PKC) and angiotensin II. Recent studies have also detected increased expression of VEGF receptors in the diabetic retina. Increased VEGF levels have been reported in the retina, aqueous humor and vitreous fluid of patients with diabetic macular edema and retinopathy. VEGF is produced by retinal pigment epithelium cells, ganglion cells, Muller cells, pericytes and smooth muscle cells of human retina and choroids.

VEGF may act directly on endothelial cell tight junctions to decrease their protein content or increase their phosphorylation, and either or both of these effects may increase paracellular permeability. The specific molecules that are allowed to move through intercellular junctions may depend on the concentration or duration of action of VEGF, as well as its interaction with other factors. It is believed that therapeutic maneuvers that suppress VEGF production or activity should be able to inhibit the development or progression both of proliferative diabetic retinopathy and diabetic macular edema, and perhaps even prevent the earlier stages of diabetic retinopathy.

Glutamate is the major excitatory neurotransmitter in the retina and is involved in neurotransmission from photoreceptors to bipolar cells and from bipolar cells to ganglion cells. Elevated levels of glutamate are implicated in neurodegeneration, and glutamate is a well-known excitotoxin associated with a number of pathologic conditions. The toxic effects of glutamate on the retina, particularly the retinal ganglion cells, are well established. In both human and experimental diabetes, vitreoretinal glutamate levels are elevated. Within three months of the onset of diabetes in the streptozotocin-induced rat model, levels of retinal glutamate increase significantly. Recent studies by Kowluru et. al have confirmed an elevation of glutamate in the retina of diabetic rats. An elevation of glutamate is associated with human diabetic patients as well. When undiluted vitreous samples from diabetic and non-diabetic patients who had undergone pars planavitrectomy were analyzed, levels of glutamate were 2.5 times greater in patients with diabetes. The receptors for glutamate have been divided into two broad categories, ionotropic that directly gate channels and metabotropic that indirectly gate changes through second messengers. There are three types of ionotropic glutamate receptors, AMPA, Kainate and NMDA. The NMDA receptor was named by the synthetic agonist that activates it, N-methyl-D-aspartate. The action of glutamate on the NMDA receptor is always excitatory. One of the major effects of glutamate and NMDA receptor interaction under pathological conditions is to increase intracellular Ca++ concentration, which sets in motion the cascade leading to eventual cell death. Increased calcium concentration has been shown to stimulate protein kinase C (PKC). Previously, brimonidine has been shown to inhibit vitreal glutamate accumulation and preserve retinal neuronal function after transient ischemia and memantine, a NMDA receptor antagonist, has been shown to prevent glutamate agonist-induced toxicity in retinal ganglion cells. It has now been found, that brimonidine and memantine reduce vitreoretinal VEGF protein levels and, as a result, blood-retinal barrier (BRB) leakage was also reduced in diabetic animals. It is believed this result is effected by the attenuation of NMDA receptor and PKC activation. As shown in the following Examples, Brimonidine may be used to attenuate elevated retinal glutamate levels in STZ-treated diabetic animals. The reduced retinal glutamate and Memantine may decrease retinal NMDA receptor activation. The reduced receptor activity might reduce PKC activity responsible for elevated ocular VEGF protein levels in diabetes and thus, attenuate vitreous/retinal VEGF protein levels in STZ-treated diabetic animals. Reduced VEGF protein levels may lead to decrease in elevated retinal BRB leakage in diabetic animals. Brimonidine and Memantine did not affect the blood glucose or body weight of the STZ-treated rats. Surprisingly, the effect of Brimonidine and Memantine to reduce vitreoretinal VEGF protein levels was not seen in non-diabetic rats.

The following references, which are representative of the art, are hereby incorporated by reference in their entirety.
Funatsu H. & Yamashita H: Drug News Perspect 15: 6633-39, 2002
Witmer, AN, Vrensen, GFJM, Van Noorden, CJF & Schlingemann, RO: Progress in retinal and eye research 22:1-29, 2003
Hammes, HP, Lin, JH, Bretzel, RG, et. al. Diabetes 47: 401-6, 1998
Funatsu, H., Yamashita, H., Nakanishi, H. et. al. Br. J. Opthalmol. 86: 311-5, 2002
Funatsu, H., Yamashita, H., Ikeda, T. et. al. Am J. Opthalmol. 133:537-44, 2002
Funatsu, H., Yamashita, H., Shimizu, E. et. al. Retina 21: 469-77, 2001
Funatsu, H., Yanashita, H., Noma, H. et. al. Am J. Opthalmol. 133:70-7, 2002
Lutty, GA, McLoad, DS, Merges, C. et. al. Arch Opthalmol 114:971-9, 1996
Antonetti, DA, Barber, AJ, Khin, S. et. al. Diabetes 47:1953-9,1998
Lucas DR & Newhouse JP. Arch Opthalmol 58:193-201, 1957
Olney JW; J. Neuropathol Exp Neurol 28: 455-74, 1969
Hansson HA, Virchows Arch 6:1-11, 1970
Lund-Karlsen RF and Fonnum F. J. Neurochem27:1437-41, 1976
Vorwerk, CK, Lipton SA, Zurakowski D, Hyman BT, abel BA and Dreyer EB. Invest Opthalmol Vis. Sci 37:1618-24, 1996
Sisk DR and Kuwabara T. Graefes arch Clin Exp Opthalmol 223:250-8, 1985
Sucker NJ, Lipton SA and Dreyer EB. Vis Res 37:3483-93, 1997
Lieth, E., Barber, AJ, Xu, B, Dice, C., Ratz, MJ, Tanase, D and Strother JM.Diabetes 47: 815-20, 1998
Howluru, RA, Engerman, RL, Case GL and Kern TS: Neurochem Int.38:385-90, 2001
Ambati J, Chalam KV, Chawla DK, D'Angio CT, Guillet EG, Rose SJ, Vanderlinde RE and Ambati BK. Arch Opthalmol 115:1161-6, 1997
Smith SB:Drug News Perspect, 15, 226-232, 2002
Xu X, Zhu Qi, Xia Xin, Zhang Shijie, Gu Qing and Luo Dawei. Current Eye Research 28, 251-256, 2004
Donello JE, OPadillo EU, Webster ML, Wheeler LA and Gil DW. J. Pharmacol and Expt. Ther. 296, 216-223, 2001
Qaum T, Xu Q, Joussen AM, Clemens MW, Qin W, Miyamoto K, Hassessian H, Wiegand SJ, Rudge J, Yancopoulos GD and Adamis AP. IOVS 42: 2408-2413, 2001.

### BRIEF SUMMARY OF THE INVENTION

The present invention provides a method of reducing the VEGF level in a patient suffering from an ocular disease or condition which is characterized by elevated VEGF and, as a result of reducing elevated VEGF, BRB leakage is also reduced, which method comprises treating said patient with an effective amount of a compound selected from the group consisting of memantine, an alpha 2 adrenergic agonist, e.g. brimonidine, and mixtures thereof. In particular, such disease or condition may be diabetic retinopathy, age-related macular degeneration (ARMD), or diabetic macular edema.

### BRIEF DESCRIPTION OF THE DRAWING FIGURES

Figure 1 shows the body weights of the rats that were used in the experiments.
Figure 2 compares the blood glucose levels of rats treated for 35days with vehicle (Veh/Veh-35D), streptozotocin (STZ-35D/Veh-28D), STZ & then memantine (Mem) after 7 days of STZ (STZ-35D/Mem-28D) or STZ & then brimonidine (Bri) after 7 days of STZ (STZ-35D/Bri-28D),
Figure 3 compares the VEGF protein levels in the retina of rats treated for 35days with vehicle (Veh/Veh-35D), STZ (STZ-35D/Veh-28D), STZ & then Mem after 7 days of STZ (STZ-35D/Mem-28D) or STZ & then Bri after 7 days of STZ (STZ-35D/Bri-28D).
Figure 4 compares the VEGF protein levels in the vitreous fluid of rats treated for 35days with vehicle (Veh/Veh-35D), STZ (STX-35D/Veh-28D), STZ & then Mem after 7 days of STZ (STZ-35D/Mem-38D) or STZ & then Bri after 7 days of STZ (STZ-35D/Bri-28D).
Figure 5 compares BRB breakdown of rats treated for 35days with vehicle (Veh/Veh-35D), STZ (STZ-35/VNeh-28D), STZ & then Mem after 7 days of STZ (STZ-35D/Mem-29D) or STZ then Bri after 7 days of STZ (STZ-35D/Bri-28D).
Figure 6 compares vitreal VEGF protein levels of non-diabetic Brown Norway (BN) rats treated for 28 days with vehicle (Veh-28D), Mem (Mem-28D), or Bri (Bri-28D).
Figure 7 compares the VEGF protein levels in the vitreous fluid of rats treated for 28 days with Vehicle, STZ and then brimonidine or various α2 Pan Agonists.
Figure 8 compares the VEGF protein levels in the vitreous fluid of rats treated for 28 days with Vehicle, STZ and then brimonidine or various α2B Agonists.

### DETAILED DESCRIPTION OF THE INVENTION

According to a further feature of the invention there is provided a pharmaceutical composition which comprises brimonidine or another Alpha 2 adrenergic agonist and/or memantine, or a phannaccutically-acceptable salt thereof, in association with a pharmaceutically-acceptable diluent or carrier for use in treating an ocular disease characterized by an increased VEGF level in the eye of a mammal, e.g. a human.

The composition may be in a form suitable for oral use, suspension or emulsion; for topical use, for example, a cream, ointment, gel, spray or aqueous or oily solution or suspension; for nasal use, for example a snuff, nasal spray or nasal drops; for vaginal or rectal use, for example a suppository or rectal spray; for administration by inhalation, for example as a finely divided powder or a liquid aerosol; for sublingual or buccal use, for example a table or capsule; or for parenteral use (including intravenous, subcutaneous, intramuscular, intra vascular or infusion), for example a sterile aqueous or oil solution or suspension. In general the above compositions may be prepared in a conventional manner using conventional excipients.

The amount of active ingredient (that is brimonidine, or other α2 adrenergic agonist, and/or memantine or a pharmaceutically-acceptable salt thereof) that is combined with one or more excipients to produce a single dosage form will necessarily vary depending upon the host treated and the particular route of administration. For example, a formulation intended for oral administration to humans will generally contain, for example, from 0.5 mg to 2 g of active agent compounded with an appropriate and convenient amount of excipients which may vary from about 5 to about 98 percent by weight of the total composition.

The dose administered to a patient may be determined by means well known in the practice of medicine.

The invention is further illustrated by the following examples which are illustrative of a specific mode of practicing the invention and are not intended as limiting the scope of the claim.

### EXAMPLE 1 - TREATMENT WITH BRIMONIDINE AND/OR MEMANTINE

Male Brown Norway (BN) rats were randomized into 4 different groups based on basal glucose and body weight. Using one time IP injection, one group of animals was treated with vehicle and other 3 groups were treated with 65 mg/kg streptozotocin (STZ). Within two days after STZ-treatment, blood glucose of the animals reached to 400 mg/dl from 100 mg/dl. After one wk of vehicle or STZ treatment, vehicle treated animals were further treated with a second vehicle for another 4 wks (Veh/Vsh-35 D) and STZ treated animals were treated with either a second vehicle (STZ-35D/Veh-28D), Memantine, Mem (10 mg/kg/day) (STZ-35D/Mem-28D) or Brimonidine, Bri (1 mg/kg/day) for 4 wks (STZ-35D/Bn-28D) using mini osmotic pumps. At end of the study after 5 wks, body weight, blood glucose, vitreoretinal VEGF protein levels and retinal BRB breakdown of the animals were measured. In some experiments, non-diabetic BN rats were treated with vehicle, Memantine (10 mg/kg/day) or Brimonidine (1 mg/kg/day) for 4 wks and at end of the study, vitreous fluid VEGF protein levels were determined.

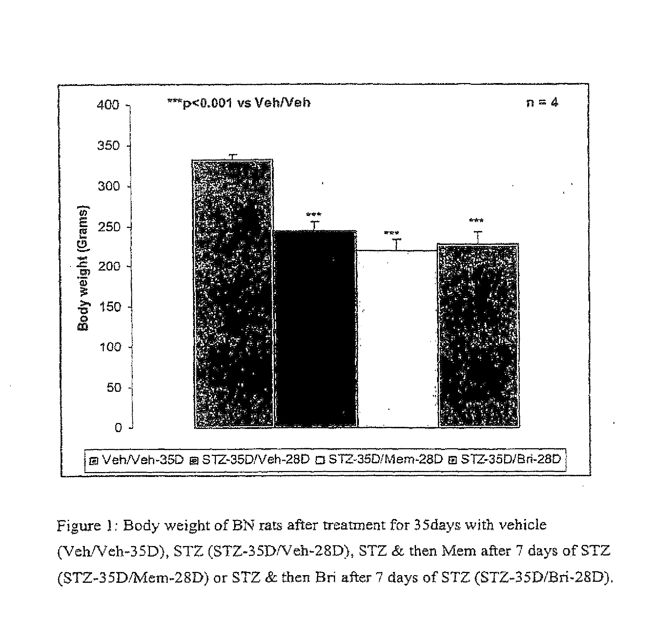

Figure 1 shows body weights of rats in different groups. Compared to vehicle treated rats (Veh/Veh-35D, 332 grams), there was a significant decrease after 5 wks in body weight of animaLs treated with STZ and then either vehicle (STZ-35D/Veh-28D, 245 grams, ***p<0.001 vs Veh/Veh-35D), Memantine (STZ-35D/Mem-28D, 219 grams, ***p<0.00 ve Veh/Veh-35D) or Brimonidine (STZ-35D/Bri-28D, 228 grams, ***p<0.00 vs Veh/Veh-35D), However, there was no difference in body weight between STZ-35DNeh-28D and STZ-35D/Mem-28D or STZ-35D/Bri-28D treated groups after 5 wks of STZ treatment.

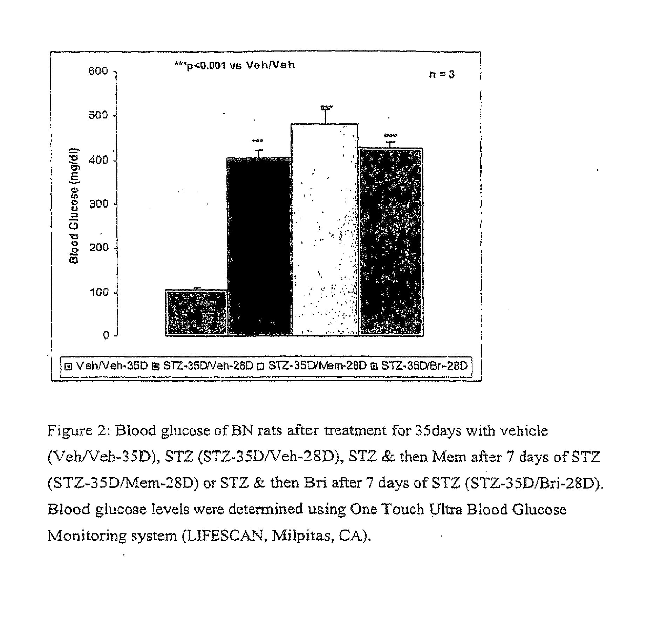

Blood glucose levels of STZ treated animals were significantly higher (> 500 mg/dl) compared to vehicle treated animals (~100 mg/dl). Mem or Bri did not have any effect on blood glucose of the animals (See Figure 2).

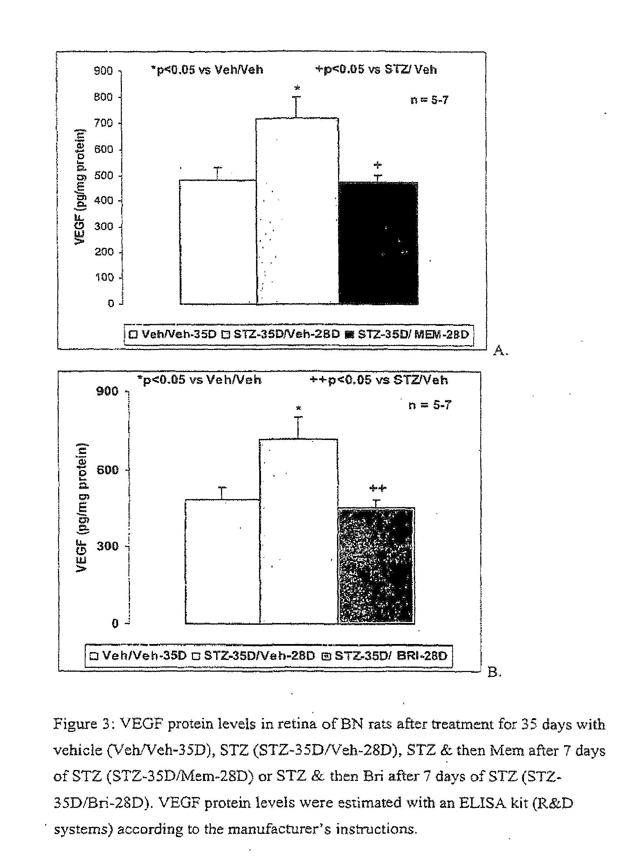

Compared to vehicle treated animals, VEGF protein levels significantly increased in retina of streptozotocin treated rats within 5 wks after treatment (STZ-35D/Veh-28D→719 pg/mg protein, Veh/Veh-35D→482 pg/mg protein, *p<0.05 vs Veh/Veh-35D)(Figure 3). However, treatment with Mem (Fig. 3A) or Bri (Fig.3B) for 4 wks after 1 wk of STZ significantly attenuated VEGF protein levels in STZ treated animals (STZ-35D/Veh-28D→19 pg/mg protein, STZ-35D/Mem-28D→475 pg/mg protein, +p<0.05 vs STZ-35D/Veh-28D & STZ-35D/Bri-28D→455 pg/mg protein, ++p< 0.05 vs STZ-35D/Veh-28D). Mem or Bri treatment brought VEGF protein levels in STZ treated animals similar to or less than Veh/Veh-35D treated animals.

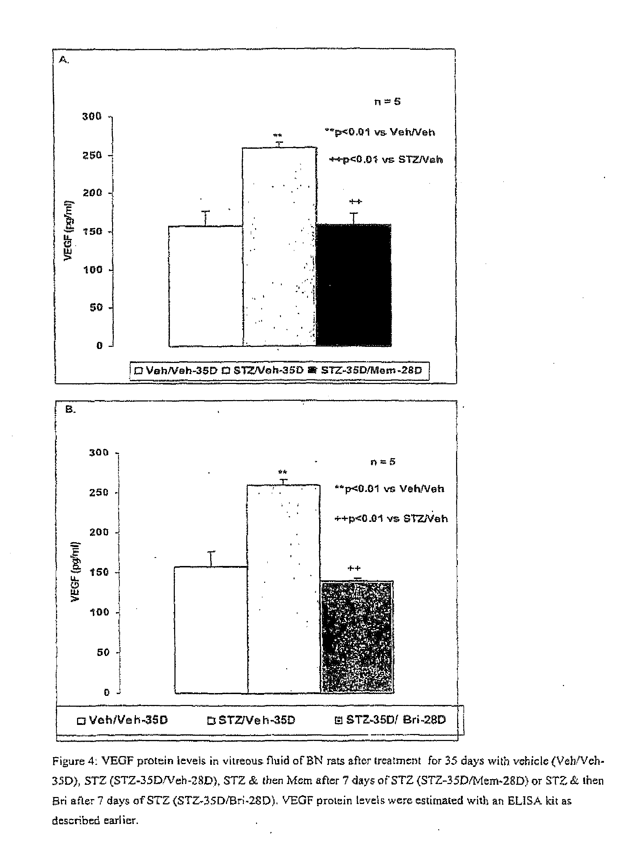

Similar to the effect on the retina, VEGF protein levels significantly increased in vitreous fluid of streptozotocin treated rats within 5 wks after treatment (STZ-35D/Veh-28D→259 pg/ml, Veh/Veh-35D→157 pg/ml, **p<0.01 vs Veh/Veh-35D)(See Figure 4). However, treatment with Mem (Fig. 4A) or Brimonidine (Figure 4B) for 4 wks after 1 wk of STZ treatment significantly attenuated VEGF protein levels in STZ treated animals (STZ-35D/Veh.28D→259 pg/ml, STZ-35D/Mem-28D→160 pg/ml, ++p< 0.01 vs STZ-35D/Veh-28D; STZ-35D/Veh-28D→259 pg/ml, STZ-35D/Bri-28→140 pg/ml, ++p< 0.01 vs STZ-35D/Veh-28D ). Mem or Bri treatment reduced VEGF protein levels in STZ treated animals to similar or less than that observed in Veh/Veh-35D treated animals.

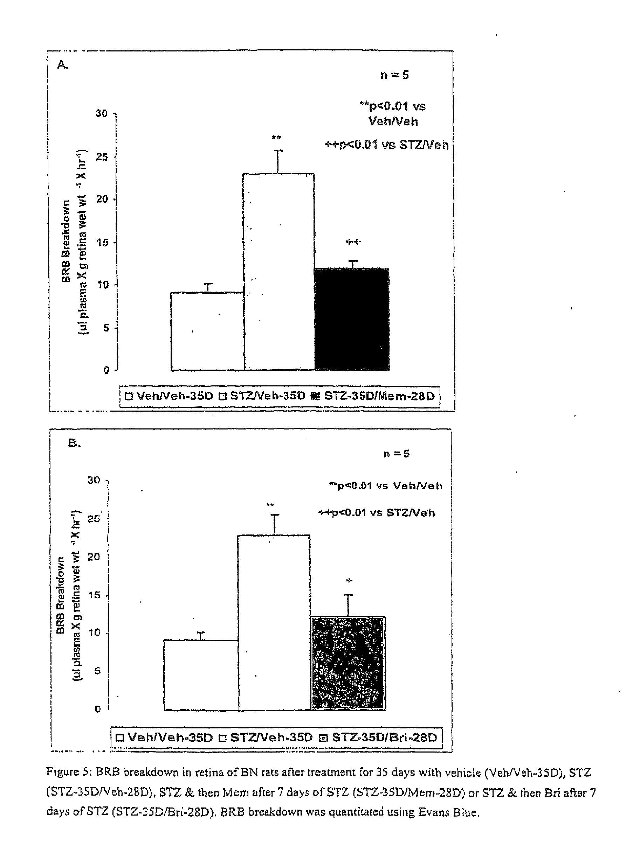

Fig. 5 shows BRB breakdown in retina of BN rats after treatment for 35 days with vehicle (Veh/Veh-35D), STZ (STZ-35D/Veh-28D), STZ & then Mem after 7 days of STZ (STZ-35D/Mem-28D) or STZ & then Bri after 7 days of STZ (STZ-35D/Bri-28D). Compared to vehicle treated animals, BRB breakdown increased significantly in retinas of STZ-treated animals within 5 wks after treatment (STZ-35D/Veh-28D; → 22.9 ul plasma/g retina dry weight/ hr & Veh/Veh-35D→9.1 ul plasma/g retina dry weight/ hr, **p<0.01 vs Veh/Veh-35D). Chronic treatment with Mem or Bri after one wk of STZ-treatment significantly reduced VEGF protein levels in these animals (STZ-35D/Veh-28D→22.9 ul plasma/g retina dry weight/ hr, STZ-35D/Mem-28D→11.9 ul plasma/g retina dry weight/ hr, ++p<0.01 vs STZ-35D/Veh-28D; STZ-35D/Veh-28D→22.9 ul plasma/g retina dry weight/ hr, STZ-35D/Bri-28D→12.2 ul plasma/g retina dry weight/ hr, +p<0.05 vs STZ-35D/Veh-28D). VEGF protein levels in Mem or Bri treated diabetic animals were similar to that of vehicle treated controls.

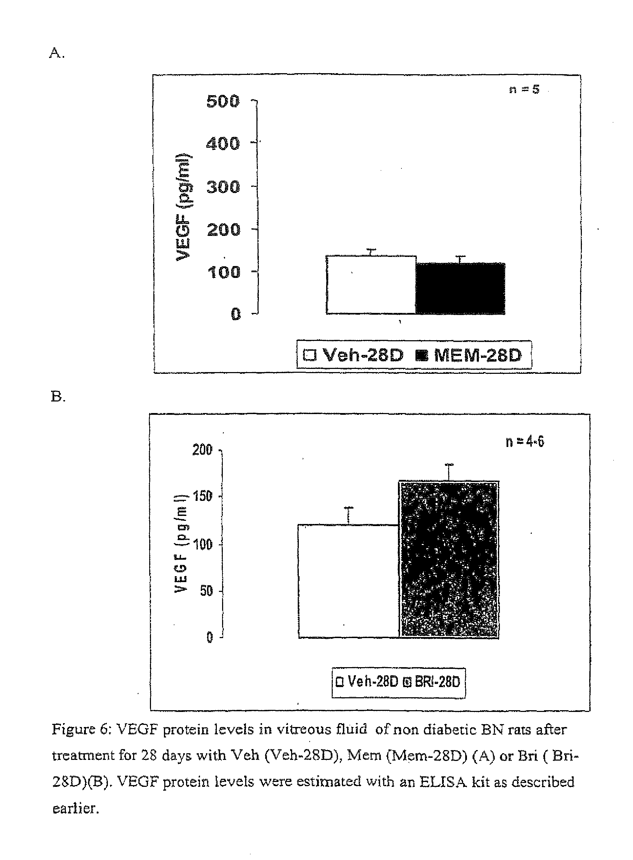

Figure 6 demonstrates VEGF protein levels in vitreous fluid of non diabetic BN rats after chronic treatment for 28 days with Veh (Veh-28D), Mem (Mem-28D) (Fig. 6A) or Bri (Bri-28D) (Fig.6B). There was no difference in vitreous fluid VEGF protein levels between vehicle and Mem or Bri-treated rats. These results suggest that unlike STZ-treated rats, chronic treatment with Mem or Bri does not affect VEGF protein levels in vitreous fluid of non-diabetic rats.

The above Examples show that, in addition to increase in blood glucose, Streptozotocin-treated diabetic rats had significantly elevated vitreoretinal VEGF protein levels and retinal BRB breakdown. Chronic treatment with Memantine or Brimonidine significantly reduces elevated VEGF protein levels in retina and vitreous fluid and retinal BRB breakdown of STZ-treated diabetic rats.

Thus, memantine and Brimonidine are useful for treatment of ocular diseases with elevated vitreoretinal VEGF protein levels and/or retinal BRB leakage. For example, such diseases include diabetic macular edema and retinopathy of prematurity as well as the other diseases noted above.

### EXAMPLE 2- TREATMENT WITH ALPHA 2 PANACONISTS

Long Evans (LE) rats were treated with vehicle or streptozotocin (1X, 65 mg/kg). After 7 days, vehicle treated rats were treated further with a second vehicle and STZ treated diabetic rats were treated either with a second vehicle, Brimonidine (1 mg/kg/d) as a positive control, Panagonist '222 (300 ug/kg/d) or '818 (300 ug or 3 mg/kg/d) for another 3 wks using mini-osmotic pumps. Note that the synthesis of these two panagonists is known in the art. The panagonists have the following structure

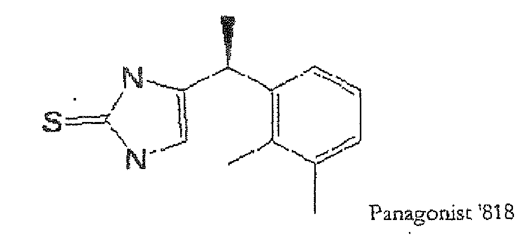

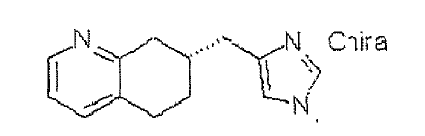

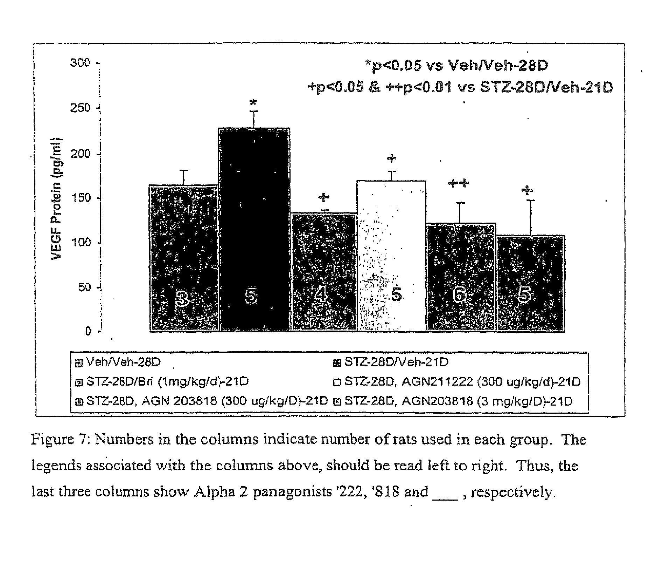

At end of the study, animals were sacrificed & vitreal VEGF protein level was measured as described earlier. The results are shown in Figure 7.

Compared to vehicle treated animals (Veh/Veh-28D), VEGF protein levels were significantly increased 4 wks after treatment in vitreous fluid of streptozotocin induced diabetic LE rats (STZ-28D/Veh-21D→ 228 pg/ml, Veh/Veh-28D→ 164 pg/ml, *p<0.01 vs Veh/Veh-28D). However, treatment with Bri for 3 wks (1 wk after STZ) significantly attenuated elevated VEGF protein levels in vitreous fluid of STZ treated animals (STZ-35D/Veh-21D→ 228 pg/ml, STZ-28D/Bri-21D→133 pg/ml, ++p<0.01 vs STZ-35D/Veh-21D). This is consistent with Example 2, above, as shown for rats. In addition, the panagonists '222 and '818 also significantly attenuated elevated vitreal VEGF protein levels in diabetic animals, and AGN 203818 inhibited in a dose dependent manner [STZ-35D/Veh-21D→ 228 pg/ml, STZ-28D '222-21D→133 pg/ml (++p<0.01 vs STZ-28D, Veh-21D), STZ-28D '818 (300 ug/kg/d)-21D→ 122 pg/ml (++p<0.01 vs STZ-28D, Veh-21D) and STZ-28D '818 (3 mg/kg/d)-21D→109 pg/ml (+p<0.01 vs STZ-35D, Veh-28D)]. Thus, STZ treatment significantly upregulated vitreous fluid VEGF protein levels. In addition to Brimonidine, chronic treatment with '222 or '818 brought elevated VEGF protein levels in vitreous fluid of STZ treated animals similar or less to levels observed in vehicle treated control animals.

### EXAMPLE 3 - TREATMENT WITH Alpha 2B SELECTIVE AGONISTS

Long Evans rats were treated with vehicle or streptozotocin (1X, 65 mg/kg). After 7 days, vehicle treated rats were treated further with a second vehicle and STZ treated diabetic rats were treated either with a second vehicle, Brimonidine (1 mg/kg/d) as a positive control, or the Alpha 2B selective agonist.

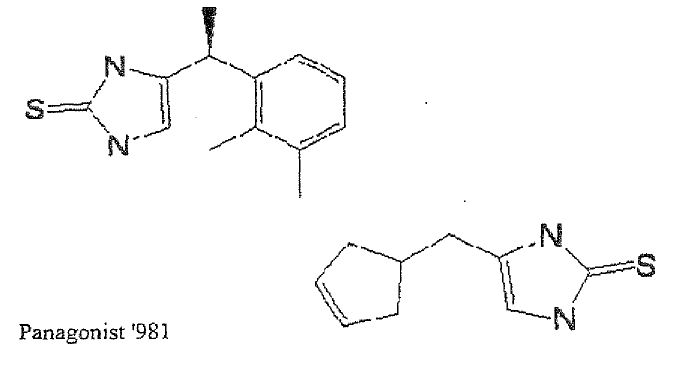

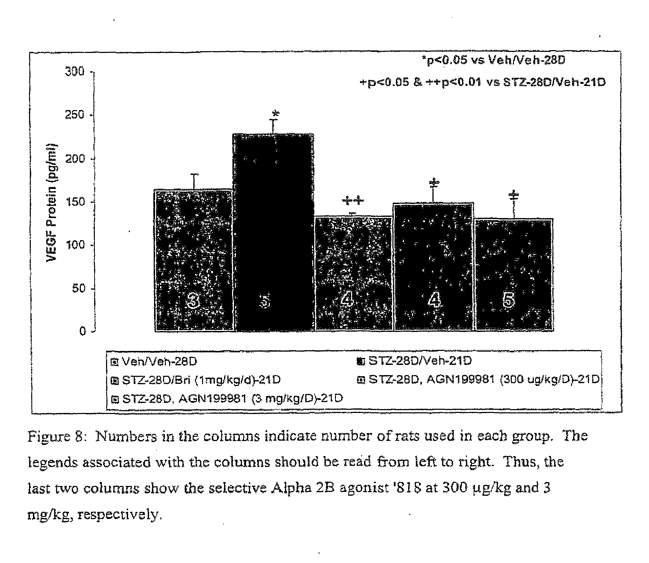

The Alpha 2B selective Agonist ("2B Agonist") (300ug or 3 mg/kg/d) for another 3 wks using mini-osmotic pumps is an Alpha 2B selective agonist. At end of the study, animals were sacrificed & vitreal VEGF protein level was measured as described earlier. The results are reported in Figure 8.

Compared to vehicle treated animals (Veh/Veh-28D), VEGF protein levels were significantly increased 4 wks after treatment in vitreous fluid of streptozotocin induced diabetic LE rats ( STZ-28D/Veh-21D→ 228 pg/ml, Veh/Veh-28D→ 164 pg/ml, *p<0.01 vs Veh/Veh-28D). However, treatment with Bri for 3 wks (1 wk after STZ) significantly attenuated elevated VEGF protein levels in vitreous fluid of STZ treated animas (STZ-28D/Veh-21D→ 228 pg/ml, STZ-28D/Bri-21D→133 pg/ml, ++p<0.01 vs STZ-28D/Veh-21D). This is consistent with the results observed in BN rats. In addition, the 2B Agonist also significantly attenuated elevated vitreal VEGF protein levels in diabetic animals in a dose dependent manner (STZ-28D/Veh-21D→ 228 pg/ml, STZ-28D/2B Agonist (300 ug/kg/d). 21D→ 148 pg/ml (+p<0.01 vs STZ-28D/Veh/21D) and STZ-28D/2B Agonist.

(3 mg/kg/d)-21D→130 pg/ml ((+p<0.01 vs STZ-28D/Veh-21D). Thus, STZ treatment significantly upregulated vitreous fluid VEGF protein levels. In addition to Brimonidine, chronic treatment with 2B Agonist brought elevated VEGF protein levels in vitreous fluid of STZ treated animals similar or less to levels observed in vehicle treated control animals.

The method of the present invention may also be used to treat the following diseases and conditions of the eye:
Nonneovascular (Nonexudative) Age-Related Macular Degeneration
Neovascular (Exudative) Age-Related Macular Degeneration
Other Causes of Choroidal Neovascular Membranes
Angioid Streaks
Ocular Histoplasmosis Myopic Retinal Degeneration
Central Serous Chorioretinopathy
Macular Hole
Epimacular Membranes
Postsurgical Cystoid Macular Edema
Retinal Arterial Occulsive Diseases
Central Retinal Vein Occlusion
Branch Retinal Vein Occlusion
Diabetic Retinopathy
Hypertensive Fundus Changes
Ocular Ischemic Syndrome
Retinal Arterial Macroaneurysms
Coats Disease
Parafoveal Telangiectasis
Retinopathy of Prematurity
Eales' Disease
Familial Exudative Vitreoretinopathy
Frosted Branch Angiitis
Hemoglobinopathies Including Sickle Cell Retinopathy
Disseminated Intravascular Coagulopathy
Radiation Retinopathy
Bone Marrow Transplant Retinopathy
Uveitic Retinal Disease
Acute Multifocal Posterior Placoid Pigment Epitheliopathy (AMPPPE)
Serpiginous Choroiditis
Birdshot Retinochoroidopathy
Sympathetic Ophthalmia
Vogt-Koyanagi-Harada Syndrome
Multiple Evanescent White Dot Syndrome (MEWDS)
Acute Macular Neuroretinopathy
Acute Retinal Pigment Epitheliitis
Intermediate Uveitis (Pars Planitis)
Multifocal Choroiditis, Punctate Inner Choroidopathy, and Other Related Uveitic Conditions
Subretinal Fibrosis and Uveitis Syndrome
Posterior Scleritis
Ocular Sarcoidosis
Toxoplasmosis
Ocular Toxocariasis
Ocular Cysticercosis
Retinol Disease Associated with HIV Infection
The Retina and Choroid in HIV Infection
Viral Retinitis
Acute Retinal Necrosis
Progressive Outer Retinal Necrosis
Fungal Diseases of the Retina
Photic Reinopathy
Drug-Induced Toxic Retinopathies
Ocular Syphilis
Tuberculosis
Diffuse Unilateral Subacute Neuroretinitis
Myiasis
Retinitis Pigmentosa and Other Related Degenerations
Systemic Genetic Disorders Associated with Retinal Dystrophies
Congenital Stationary Night Blindness
Cone Dystrophies
Stargardt's Disease and Fundus Flavimaculatus
Best's Disease: Molecular and Clinical Findings
Pattern Dystrophy of the Retinal Pigment Epithelium
X-Linked Retinoschisis
Sorsby's Fundus Dystrophy
Benign Concentric Maculopathy
Bietti's Crystalline Dystrophy
Tumors
Retinal Disease Associated with Tumors Congenital Hypertrophy of the Retinal Pigment Epithelium
Posterior Uveal Melanoma Differential Diagnosis of Posterior Uveal Melanoma
Choroidal Hemangioma
Choroidal Osteoma
Choroidal Metastasis
Combined Hamartoma of the Retina and Retinal Pigment Epithelium
Retinoblastoma
Vasoproliferative Tumors of the Ocular Fundus including Von-Hippel Lindau disease
Retinal Astrocytoma
Intraocular Lymphoid Tumors
Endophthalmitis Management
Retinal Detachment

The use of the above alpha 2 adrenergic agonists and/or memantine is distinct from the neuroprotective effects of these compounds. Furthermore, although this invention has been exemplified by the use of brimonidine and other proprietary alpha panagonists and/or alpha 2 B-selective agonists to treat such diseases of the eye, other alpha 2 agonists, for example, clonidine, guanfacine, BHT-920, para-amino clonidine (PAC), guanabenz, oxymetazoline, xylometazoline, xylazine, tizanidine, dexmedetomidine, medetomidine, mivazerol and moxonidine, etc. may be utilized in place of brimonidine to treat diseases of the eye by reducing VEGF and thereby preventing and/or reducing BRB.

Also, while this invention has been exemplified by the use of memantine to treat such diseases of the eye, other NMDA antagonists, for example ifenprodil, Dextromethorphan, Ketamine, amantadine and Kynurenate, etc. may be utilized in place of memantine.
1. A method of reducing the VEGF level in a patient suffering from an ocular disease or condition which is characterized by elevated VEGF and/or blood retina barrier (BRB) breakdown which comprises treating said patient with an effective amount of a compound selected from the group consisting of memantine, brimonidine and mixtures thereof.
2. The method of EM 1 wherein said compound is memantine.
3. The method of EM 1 wherein said compound is brimonidine.
4. A method of reducing the VEGF level in a patient suffering from an ocular disease or condition which is characterized by elevated VEGF and/or BRB breakdown which comprises treating said patient with an alpha 2 agonist.
5. The method of EM 4 wherein said alpha 2 agonists comprises clonidine, guanfacine, BHT-920, para-amino clonidine (PAC), guanabenz, oxymetazoline, xylometazoline, xylazine, tizanidine, dexmedetomidine, medetomidine, mivazerol and moxonidine.
6. The method of EM 1 wherein said ocular disease or condition is characterized by elevated VEGF in the vitreous fluid.
7. The method of EM 1 wherein said ocular disease or condition is selected from the group consisting of proliferative diabetic retinopathy, age-related macular degeneration, diabetic macular edema, tumors, wound healing or inflammation.
8. A method of reducing the VEGF level in a patient suffering from an ocular disease or condition which is characterized by elevated VEGF and/or BRB breakdown which comprises treating said patient with an NMDA antagonist.
9. The method of EM 8 wherein said NMDA antagonist is selected from the group consisting of ifenprodil, Dextromethorphan, Ketamine, amantadine and Kynurenate.
10. The method of is EM 4 wherein said alpha 2 agonist is a alpha 2 panagonist.
11. The method of EM 10 wherein said alpha 2 panagonist is selected from the group consisting of 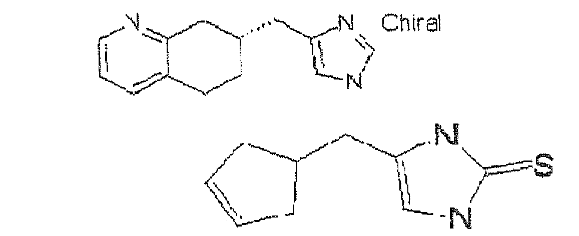
12. The method of EM 4 wherein said alpha 2 agonist is a selective alpha 2B agonist.
13. The method of EM 12 wherein said alpha 2B agonist is

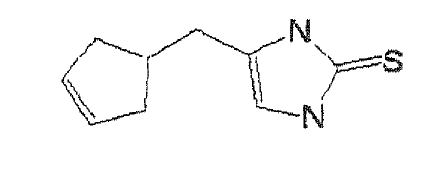

## Claims

1. An NDMA antagonist for use in a method of reducing or preventing blood-retina barrier (BRB) breakdown in a patient which comprises treating said patient with an effective amount of the NDMA antagonist.

2. The NDMA antagonist of claim 1 which is ifenprodil, dextromethorphan, ketamine, amantadine or kynurenate.

3. The NDMA antagonist of claim 1, wherein the BRB breakdown is a result of elevated VEGF in the vitreous fluid.

4. The NDMA antagonist of claim 1, wherein the BRB breakdown is a result of an ocular disease or condition selected from the group consisting of proliferative diabetic retinopathy, age-related macular degeneration, diabetic macular edema, tumors, wound healing or inflammation.
